(19) Europäisches Patentamt
European Patent Office
Office européen des brevets

(11) Numéro de publication: **0 490 769 B1**

(12) **FASCICULE DE BREVET EUROPEEN**

(45) Date de publication de fascicule du brevet: **19.04.95**

(51) Int. Cl.[6]: **C07D 333/22**, C07D 409/06, A61K 31/40, A61K 31/445, A61K 31/535, A61K 31/41, A61K 31/55

(21) Numéro de dépôt: **91403374.1**

(22) Date de dépôt: **12.12.91**

(54) **Nouveaux dérivés d'aminoalkycétone, leur procédé de préparation et leurs applications en thérapeutique.**

(30) Priorité: **12.12.90 FR 9015591**

(43) Date de publication de la demande:
**17.06.92 Bulletin 92/25**

(45) Mention de la délivrance du brevet:
**19.04.95 Bulletin 95/16**

(84) Etats contractants désignés:
**AT BE CH DE DK ES FR GB GR IT LI LU NL SE**

(56) Documents cités:
**EP-A- 0 193 875**
**DE-A- 2 552 151**
**FR-A- 2 134 218**
**FR-A- 2 453 172**
**FR-M- 3 414**

(73) Titulaire: **LABORATOIRE L. LAFON**
**19 Avenue du Professeur Cadiot**
**F-94701 Maisons Alfort (FR)**

(72) Inventeur: **Lafon, Louis**
**5, rue de l'Alboni**
**F-75016 Paris (FR)**

(74) Mandataire: **Le Guen, Gérard et al**
**CABINET LAVOIX**
**2, place d'Estienne d'Orves**
**F-75441 Paris Cédex 09 (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

La présente invention concerne de nouveaux dérivés d'aminoalkylcétone, leur procédé de préparation et leurs applications en thérapeutique, notamment comme vasodilatateurs périphériques.

Dans FR-A-2 134 218 on a décrit des dérivés d'aminoalkylcétone ayant une activité vasodilatatrice périphérique, et notamment le buflomédil.

La présente invention vise à fournir de nouveaux composés qui non seulement ont une activité vasodilatatrice périphérique, mais également un activité intéressante sur le métabolisme.

La présente invention a ainsi pour objet des composés de formule

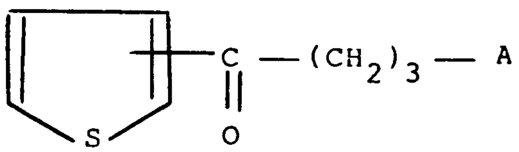

(I)

dans laquelle A est choisi parmi les groupes pyrrolidino, pipéridino, morpholino 1-imidazolyle, hexaméthylène imino, 1-pipérazinyle, ces groupes étant non substitués ou pouvant comporter 1 ou 2 substituants choisis parmi les groupes alkyle en $C_1$-$C_3$ et hydroxyalkyle en $C_1$-$C_3$,
et les sels d'addition de ces composés avec des acides pharmaceutiquement acceptables.

Comme exemples de groupes A, on peut citer les groupes pyrrolidino, 2,4-diméthyl pyrrolidino, 2,5-diméthyl pyrrolidino, pipéridino, 3-méthyl pipéridino, 3-hydroxyméthyl pipéridino, morpholino, 1-imidazolyle, hexaméthylène imino et N-méthylpipérazino.

Les "sels d'addition avec des acides pharmaceutiquement acceptables" désignent les sels qui donnent les propriétés biologiques des bases libres, sans avoir d'effet indésirable. Ces sels peuvent être notamment ceux formés avec des acides minéraux, tels que l'acide chlorhydrique, l'acide bromhydrique, l'acide sulfurique, l'acide nitrique, l'acide phosphorique ; des sels métalliques acides, tels que l'orthophosphate disodique et le sulfate monopotassique, et des acides organiques, tels que l'acide formique, l'acide acétique, l'acide propionique, l'acide glycolique, l'acide oxalique, l'acide fumarique, l'acide maléique, l'acide citrique, l'acide malonique, l'acide méthane sulfonique, l'acide lactique, l'acide succinique, l'acide tartrique.

Les composés selon la présente invention peuvent être préparés par condensation d'un dérivé chloré de formule :

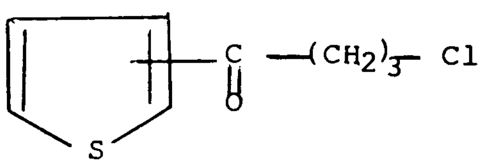

(II)

avec une amine cyclique de formule :

HA (III)

A ayant la signification donnée ci-dessus.

Cette réaction peut être effectuée dans les solvants habituellement utilisés pour les réactions de condensation.

En variante, les composés selon l'invention peuvent être préparés par réaction d'un nitrile de formule :

$NC-(CH_2)_3-A$ (IV)

avec un dérivé lithié du bromo-2 ou 3-thiophène, puis hydrolyse.

Cette réaction peut être réalisée dans les conditions classiques de mise en oeuvre des dérivés lithiés.

Les sels peuvent être obtenus de façon classique par réaction d'un composé de formule I avec un acide pharmaceutiquement acceptable dans un solvant approprié.

Les exemples suivants illustrent la préparation des composés selon l'invention.

Exemple 1

Préparation de la 4-pyrrolidino-1-(2-thiényl)butanone, chlorhydrate (CRL 41687).

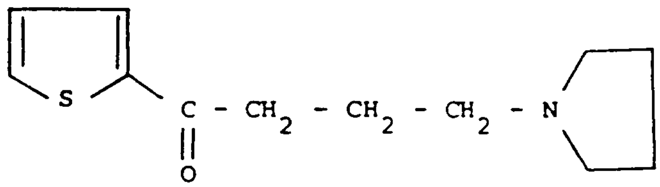

, HCl

Au sein d'une solution maintenue à 100° C de 18,5 ml (0,22 mole) de pyrrolidine dans 35 ml de toluène, on coule en 30 mn 18,85 g (0,10 mole) de 4-chloro-1-(2-thiényl)butanone et on chauffe au reflux 2 heures. On dilue le milieu réactionnel par de l'éther éthylique que l'on lave par de l'eau. La phase organique est extraite par une solution d'acide chlorhydrique 2 N qui, après alcalinisation par de la soude, est extraite à son tour par de l'éther éthylique. On sèche la phase organique sur sulfate de sodium sec et on la traite par de l'isopropanol chlorhydrique.

Le précipité obtenu est purifié par deux cristallisations successives avec traitement au noir CXA dans l'isopropanol et l'éthanol absolu pour donner 11,1 g d'une poudre légèrement grise soluble dans l'eau à 20 %.

| | |
|---|---|
| $F_{\cdot inst.}$ (Kofler) | = 177° C. |
| Rendement | = 46,3 %. |

Exemple 2

Préparation de la 4-pipéridino-1-(2-thiényl) butanone, chlorhydrate (CRL 41696).

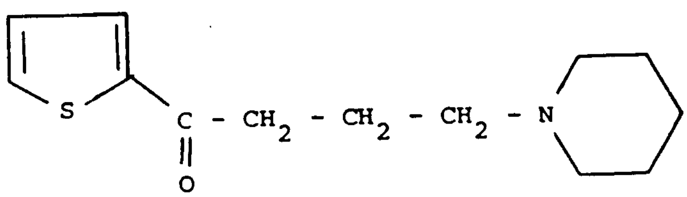

, HCl

Au sein d'une solution au reflux de 18,7 g (0,22 mole) de pipéridine dans 35 ml de toluène, on coule en 30 mn 18,9 g (0,10 mole) de 4-chloro-1-(2-thiényl)butanone et on maintient le chauffage 2 heures. On dilue le milieu réactionnel par de l'éther éthylique que l'on lave par de l'eau. La phase organique est extraite par une solution d'acide chlorhydrique dilué et la phase aqueuse alcalinisée par de la soude. On extrait par de l'éther éthylique et après séchage sur sulfate de sodium sec, on traite par de l'isopropanol chlorhydrique.

Le précipité obtenu par filtration est purifié par une cristallisation dans l'éthanol absolu pour donner : 22,5 g d'une poudre légèrement beige soluble dans l'eau.

| | |
|---|---|
| $F_{\cdot inst.}$ (Kofler) | = 178° C. |
| Rendement | = 82,3 %. |

Exemple 3

Préparation de la 4-(3-méthylpipéridino)-1-(2-thiényl) butanone, chlorhydrate (CRL 41686).

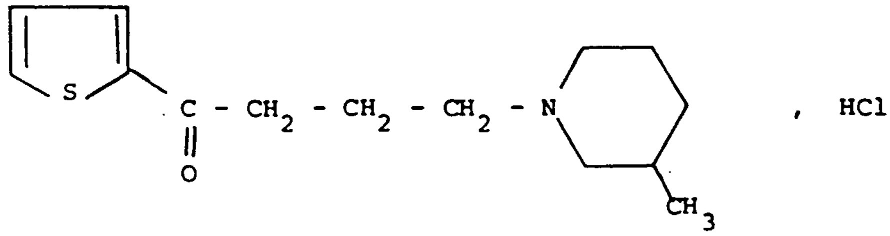

Au sein d'une solution maintenue à 100° C, de 21,8 g (0,22 mole) de méthyl-3 pipéridine dans 35 ml de toluène, on coule en 30 mn 18,85 g (0,10 mole) de 4-chloro-1-(2-thiényl)butanone et on chauffe au reflux 2 heures. On dilue le milieu réactionnel par de l'éther éthylique que l'on lave par de l'eau. La phase organique est extraite par une solution d'acide chlorhydrique diluée, qui après alcalinisation par de la soude est extraite à son tour par de l'éther éthylique. On sèche la phase organique sur sulfate de sodium sec et on la traite par de l'isopropanol chlorhydrique.

Le précipité obtenu est purifié par une cristallisation dans l'isopropanol pour donner 18 g d'une poudre blanche soluble dans l'eau à 20 %.

| | |
|---|---|
| $F_{.inst.}$ (Kofler) | = 164° C. |
| Rendement | = 62,6 %. |

Exemple 4

Préparation de la 4-morpholino-1-(2-thiényl) butanone, chlorhydrate (CRL 41698).

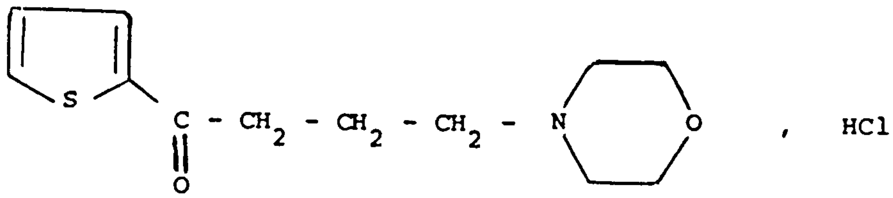

Au sein d'une solution au reflux de 19,2 g (0,22 mole) de morpholine dans 35 ml de toluène, on coule en 15 mn 18,9 g (0,20 mole) de 4-chloro-1-(2-thiényl)butanone et on poursuit le chauffage 2 heures. On dilue le milieu réactionnel par de l'éther éthylique que l'on lave par de l'eau. La phase organique est extraite par une solution d'acide chlorhydrique dilué et la phase aqueuse alcalinisée par de la soude. On extrait par de l'éther éthylique et après séchage sur sulfate de sodium sec, on traite par de l'isopropanol chlorhydrique.

Le précipité obtenu par filtration est purifié par une cristallisation dans le méthanol pour donner : 12,7 g d'une poudre beige, soluble dans l'eau.

| | |
|---|---|
| $F_{.inst.}$ (Kofler) | = 210° C. |
| Rendement | = 46,1 %. |

Exemple 5

Préparation de la 4-(3-hydroxyméthyl pipéridino)-1-(2-thiényl) butanone, chlorhydrate (CRL 41697).

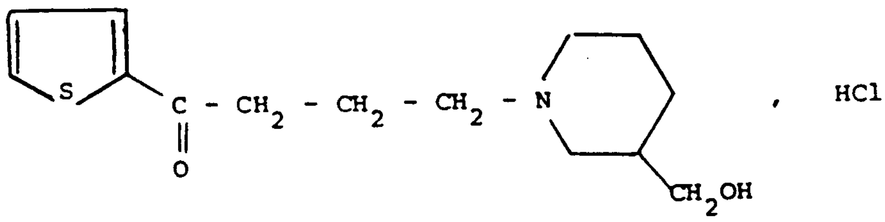

Au sein d'une solution au reflux de 38 g (0,33 mole) de 3-pipéridinyl méthanol dans 55 ml de toluène, on coule en 30 mn 28,3 g (0,15 mole) de 4-chloro-1-(2-thiényl) butanone et on maintient le chauffage 2 h 30. On dilue le milieu réactionnel par de l'acétate d'éthyle que l'on lave par de l'eau. La phase organique est extraite par une solution d'acide chlorhydrique dilué et la phase aqueuse alcalinisée par de la soude. On extrait par de l'acétate d'éthyle et après séchage sur sulfate de sodium sec, on traite par de l'isopropanol chlorhydrique.

Le précipité obtenu par filtration est purifié par une cristallisation dans l'éthanol pour donner 26 g d'une poudre légèrement grise soluble dans l'eau.

| | |
|---|---|
| $F_{.inst.}$ (Kofler) | = 160° C. |
| Rendement | = 57,1 %. |

Exemple 6

Préparation de la 4(1-imidazolyl)-1-(2-thiényl) butanone, chlorhydrate (CRL 41703).

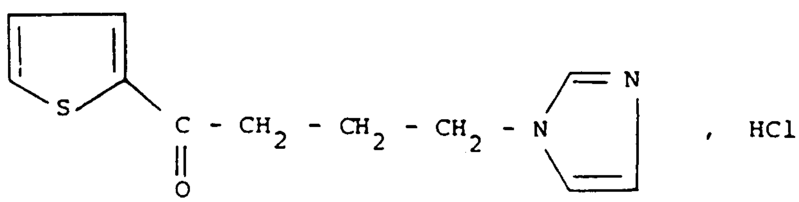

Au sein d'une solution maintenue vers 90° C de 15 g (0,22 mole) d'imidazole dans 35 ml de toluène, on coule 18,9 g (0,10 mole) de 4-chloro-1-(2-thiényl) butanone et on chautte au reflux 4 heures. On dilue le milieu réactionnel par de l'acétate d'éthyle que l'on lave par de l'eau et on extrait par une solution d'acide chlorhydrique dilué. La phase aqueuse est alcalinisée par de la soude, extraite par de l'acétate d'éthyle que l'on sèche sur sulfate de sodium sec. La solution est traitée par de l'éthanol chlorhydrique et l'insoluble purifié par un lavage dans l'acétone pour donner 6 g d'une poudre beige soluble dans l'eau.

| | |
|---|---|
| $F_{.inst.}$ (Kofler) | = 132° C. |
| Rendement | = 23,4 %. |

Exemple 7

Préparation de la 4-hexaméthylène imino-1-(2-thiényl) butanone, chlorhydrate (CRL 41683).

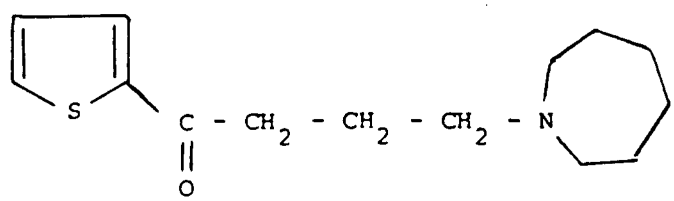

, HCl

Au sein d'une solution maintenue à 100° C de 32,7 g (0,33 mole) d'hexaméthylène imine dans 50 ml de toluène, on coule en 30 mn 28,3 g (0,15 mole) de 4-chloro-1-(2-thiényl) butanone. On poursuit le reflux 3 heures, on dilue le milieu réactionnel par 150 ml d'éther éthylique, on élimine l'insoluble par filtration et on extrait le filtrat par une solution d'acide chlorhydrique dilué. La phase aqueuse est alcalinisée par de la soude concentrée et extraite à son tour par de l'éther éthylique. Après séchage de la phase organique sur sulfate de sodium sec, on la traite par de l'isopropanol chlorhydrique.

Le précipité est isolé par filtration puis purifié par une cristallisation avec traitement au noir CXA dans l'éthanol absolu pour donner 33,1 g d'une poudre beige soluble dans l'eau.

| | |
|---|---|
| $F_{\cdot inst.}$ (Kofler) | = 181° C. |
| Rendement | = 76,75 %. |

Exemple 8

Préparation de la 4-pyrrolidino-1-(3-thiényl) butanone, chlorhydrate (CL 41724).

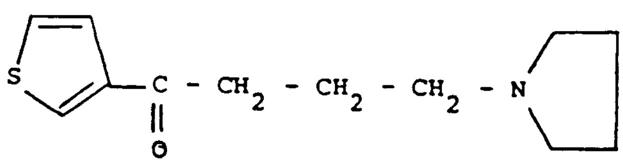

, HCl

Au sein d'une solution sous atmoshère d'azote maintenue à -70° C de 69 ml (0,11 mole) de butyl lithium en solution 1,6 M dans l'hexane diluée dans 150 ml d'éther éthylique, on coule en 10 mn une solution de 16,3 g (0,10 mole) de 3-bromo thiophène dans 50 ml d'éther éthylique.

En maintenant à -50° C, on coule en 15 mn, 12,5 g de 4-pyrrolidino butyronitrile et on agite 2 heures en laissant revenir à la température ambiante. On jette le milieu réactionnel sur 85 g de glace et 42,5 ml d'acide chlorhydrique 12 N, on agite 1 heure, on décante la phase aqueuse que l'on alcalinise par de la soude et qu'on extrait par de l'éther éthylique.

La phase organique est lavée par de l'eau, séchée sur sulfate de sodium sec puis traitée par de l'isopropanol chlorhydrique.

Le précipité obtenu est purifié par une cristallisation avec traitement au noir CXA dans le mélange acétate d'éthyle - 125/isopropanol - 40, pour donner 15 g d'une poudre beige soluble dans l'eau.

| | |
|---|---|
| $F_{\cdot inst.}$ (Kofler) | = 124° C. |
| Rendement | = 64,2 %. |

Exemple 9

Préparation de la 4-hexaméthylène imino-1-(3-thiényl) butanone, chlorhydrate (CRL 41725).

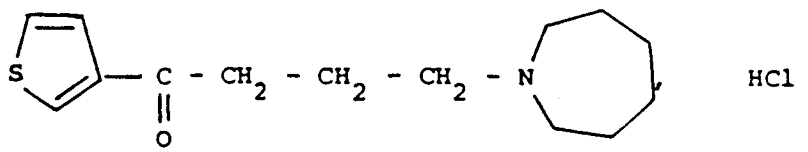

Au sein d'une solution sous atmosphère d'azote maintenue à -70° C de 69 ml (0,11 mole) de butyllithium en solution 1,6 M dans l'hexane, diluée dans 150 ml d'éther éthylique, on coule en 15 mn une solution de 16,3 g (0,10 mole) de 3-bromo-thiophène dans 50 ml d'éther éthylique.

En maintenant -50° C, on coule en 10 mn 16,6 g (0,10 mole) d'hexaméthylène imino butyronitrile et on agite 4 heures en laissant revenir à la température ambiante. On jette le milieu réactionnel sur 85 g de glace et 42,5 ml d'acide chlorhydrique 12 N, on agite 1 heure, on décante le phase aqueuse que l'on alcalinise par de la soude et on extrait par de l'éther éthylique.

La phase organique est lavée par de l'eau, séchée sur sulfate de sodium sec puis traitée par de l'isopropanol chlorhydrique.

Le précipité obtenu est purité par une cristallisation avec traitement au noir CXA dans l'éthanol absolu pour donner 19,5 g d'une poudre légèrement grise, soluble dans l'eau.

| | |
|---|---|
| $F_{\cdot inst.}$ (Kofler) | = 200-202° C. |
| Rendement | = 67,8 %. |

Exemple 10

Préparation de la 4-(3-méthylpipéridino)-1-(3-thiényl) butanone, chlorhydrate (CRL 41726).

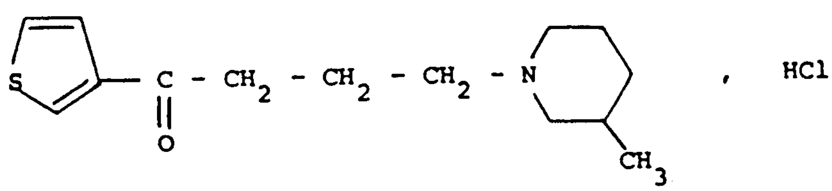

a) Préparation du 4-(3-méthylpipéridino) butyronitrile.

Au sein d'une solution au reflux de 58,8 ml (0,50 mole) de 3-méthyl pipéridine dans 65 ml de benzène, on coule en 25 mn 27,2 g (0,25 mole) de 4-chloro butyronitrile. On poursuit le reflux 2 h, on élimine le précipité par filtration et on amène 1 filtrat à siccité sous pression réduite.

Le résidu est purifié par une distillation sous pression réduite pour donner 29,2 g d'une huile incolore.

| | |
|---|---|
| $E_{5-6\ mm}$ | = 100°. |
| Rendement | = 70,3 %. |

b) Préparation de la 4-(3-méthylpipéridino)-1-(3-thiényl) butanone.

Au sein d'une solution sous atmosphère d'azote maintenue à -70° C de 69 ml (0,11 mole) de butyl lithium en solution 1,6 M dans l'hexane diluée dans 150 ml d'éther éthylique, on coule en 10 mn une

solution de 16,3 g (0,10 mole) de 3-bromo thiophène dans 50 ml d'éther éthylique.

En maintenant -50° C, on coule en 15 mn 16,6 g (0,10 mole) du produit obtenu en a) et on agite 2 heures en laissant revenir à la température ambiante. On jette le milieu réactionnel sur 85 g de glace et 42,5 ml d'acide chlorhydrique 12 N, on agite 1 heure, on décante la phase aqueuse que l'on alcalinise par de la soude et on extrait par de l'éther éthylique.

La phase organique est lavée par de l'eau, séchée sur sulfate de sodium sec puis traitée par de l'isopropanol chlorhydrique.

Le précipité obtenu est purifié par une cristallisation avec traitement au noir CXA dans l'éthanol absolu, pour donner 16,1 g d'une poudre légèrement rose soluble dans l'eau.

| | |
|---|---|
| $F_{\cdot inst.}$ (Kofler) | = 184° C. |
| Rendement | = 56 % |
| Rendement total | = 39,4 %. |

Exemple 11

Préparation de la 4-pyrrolidino-1-(2-thiényl) butanone, 2,4,6-trimétoxybenzoate (CRL 41784)

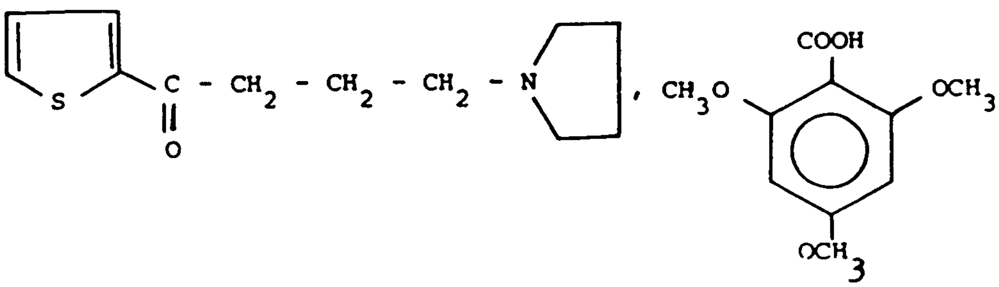

On mélange 9,9 g (0,044 mole) de 4-pyrrolidino-1-(2-thiényl)butanone et 9,4 g (0,044 mole) d'acide 2,4,6-triméthoxybenzoïque dans 50 ml d'acétone. On agite 1 heure à la température ambiante, on dilue le milieu réactionnel par 25 ml d'acétone et on porte au reflux pour dissoudre.

Après refroidissement, on isole par filtration : 12,3 g d'une poudre beige soluble dans l'eau à chaud.

| | |
|---|---|
| $F_{\cdot inst.}$ (Kofler) | # 115°C |
| Rendement | : 64,2 %. |

Exemple 12

Préparation de la 4-pipéridino-1-(2-thiényl) butanone, 2,4,6-triméthoxybenzoate (CRL 41777)

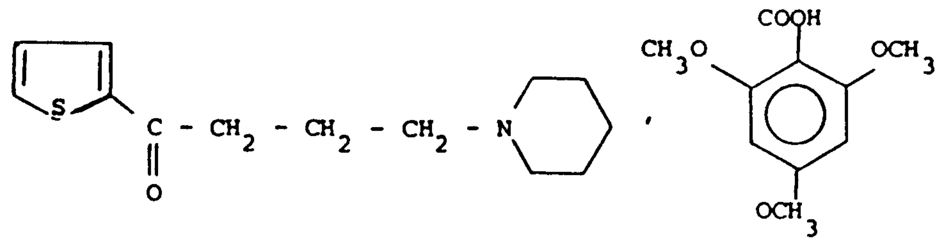

Au sein d'une solution de 10 g (0,0365 mole) de 4-pipéridino-1-(2-thiényl) butanone dans 50 ml de méthanol, on introduit 7,7 g (0,0365 mole) d'acide 2,4,6-triméthoxy benzoïque. On agite 30 mn à la température ambiante, on amène le milieu réactionnel à siccité et on reprend le résidu par de l'acétone. La solution est insolubilisée par de l'éther éthylique et le précipité isolé par filtration.

Ce produit est purifié par un lavage dans l'acétone, puis par une cristallisation avec traitement au noir CXA dans l'acétone, pour donner 6,8 g d'une poudre blanche soluble dans l'eau à chaud.

| | |
|---|---|
| F | # 100 ° C(fusion pâteuse) |
| Rendement | = 41,5 %. |

On donnera ci-après des résultats pharmacologiques et toxicologiques mettant en évidence les propriétés intéressantes des composés selon l'invention.

**a) Toxicité aigüe**

La toxicite aigüe a été déterminée par voie orale chez la souris (NMRI).

| Composé | DL 50 (mg/kg) |
|---|---|
| Exemple 1 | 121 ± 10 |
| Exemple 2 | 235 ± 35 |
| Exemple 3 | 207 ± 29 |
| Exemple 7 | 131 ± 15 |

**b) Activité cardiovasculaire**

On a étudié l'activité des composés chez le chien anesthésié par administration intraveineuse.

Composé de l'exemple 1 (CRL 41687)

Ce composé augmente de façon notable le débit fémoral dès la dose de 1 mg/kg. A la dose de 4 mg/kg, il augmente de 170 % le débit fémoral.

A titre de comparaison, le buflomédil à la dose de 4 mg/kg augmente le débit fémoral de 106 %.

Dans le même essai, on n'observe pas de variation notable de la pression artérielle avec le composé de l'exemple 1, même à la dose de 4 mg/kg.

Composé de l'exemple 2 (CRL 41696)

Ce composé augmente de façon notable le débit fémoral dès la dose de 1 mg/kg. A la dose de 2 mg/kg, il augmente le débit fémoral avec la même intensité que le buflomédil à la dose de 4 mg/kg, sans modifier la pression artérielle. L'effet du composé de l'exemple 2 est plus durable que celui du buflomédil.

Composé de l'exemple 7 (CRL 41683)

Ce composé augmente de façon notable et durable le débit fémoral dès la dose de 1,14 mg/kg. Cette augmentation est plus importante que celle observée avec le buflomédil à la dose de 4 mg/kg.

**c - Activité sur la microcirculation**

On a étudié l'activité des composés par voie intraveineuse sur la microcirculation de la chambre de l'oreille du lapin.

A la dose de 1 mg/kg I.V. les composés des exemples 1 et 7 (CRL 41687 et CRL 41683) augmentent de façon notable et durable le diamètre artériolaire non terminal.

**d - Action sur le métabolisme**

On a mesuré les effets des composés sur l'ATP du sang frais ex vivo par spectroscopie RMN du $^{31}P$.

Les résultats sont donnés dans le tableau ci-après sous forme de pourcentage de variations.

| Composé | $\gamma$ ATP | $\alpha$ ATP | $\beta$ ATP |
|---|---|---|---|
| Ex. 1 | + 36,7 % | + 41,4 % | + 31,2 % |
| Ex. 3 | + 34,7 % | + 54,2 % | + 54,6 % |

Les mêmes essais effectués avec le buflomédil n'ont pas mis en évidence de variation du taux d'ATP.

La présente invention a également pour objet des compositions thérapeutiques comprenant à titre de principe actif un composé de formule I ou l'un de ses sels d'addition avec des acides pharmaceutiquement acceptables.

Les compositions thérapeutiques selon l'invention peuvent être administrées à l'homme ou aux animaux par voie orale ou parentérale.

Elles peuvent être sous la forme de préparations solides, semi-solides ou liquides. Comme exemple, on peut citer les comprimés, les gelules, les suppositoires, les solutions ou suspensions injectables, ainsi que les formes-retard et les formes implantées à libération lente.

Dans ces compositions, le principe actif est généralement mélangé avec un ou plusieurs excipients pharmaceutiquement acceptables habituels bien connus de l'homme de l'art.

La quantité de principe actif administrée dépend évidemment du patient qui est traité, de la voie d'administration et de la sévérité de la maladie.

En général, on peut administrer par voie orale les composés selon l'invention à une dose de 100 à 800 mg/jour.

**Revendications**

**Revendications pour les Etats contractants suivants : AT, BE, CH, DE, DK, FR, GB, GR, IT, LI, LU, NL, SE**

1. Composés de formule :

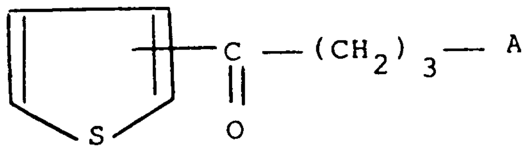

(I)

dans laquelle A est choisi parmi les groupes pyrrolidino, pipéridino, morpholino, 1-imidazolyle, hexaméthylène imino, 1-pipérazinyle, ces groupes étant non substitués ou pouvant comporter 1 ou 2 substituants choisis parmi les groupes alkyle en $C_1$-$C_3$ et hydroxyalkyle en $C_1$-$C_3$, et les sels d'addition de ces composés avec des acides pharmaceutiquement acceptables.

2. Composés selon la revendication 1, dans lequels A est un groupe pyrrolidino.

3. Composés selon la revendication 1, dans lesquels A est choisi parmi un groupe pipéridino, 3-méthylpipéridino, 3-hydroxyméthylpipéridino.

4. Composés selon la revendication 1 qui sont choisis parmi la 4-pyrrolidino-1-(2-thiényl) butanone et ses sels d'addition avec des acides pharmaceutiquement acceptables.

5. Composition thérapeutique comprenant à titre de principe actif un composé selon l'une quelconque des revendications 1 à 4.

6. Utilisation d'un composé selon l'une quelconque des revendications 1 à 4 pour la fabrication d'un médicament à activité vasodilatatrice.

**Revendications pour l'Etat contractant suivant : ES**

1. Procédé de préparation de composés de formule :

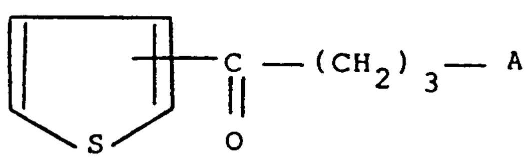

(I)

dans laquelle A est choisi parmi les groupes pyrrolidino, pipéridino, morpholino, 1-imidazolyle, hexaméthylène imino, 1-pipérazinyle, ces groupes étant non substitués ou pouvant comporter 1 ou 2 substituants choisis parmi les groupe alkyle en $C_1$-$C_3$ et hydroxyalkyle en $C_1$-$C_3$, et les sels d'addition de ces composés avec des acides pharmaceutiquement acceptables,
comprenant la condensation d'un dérivé chloré de formule:

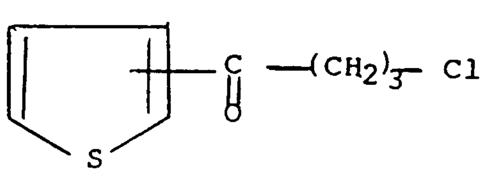

(II)

avec une amine cyclique de formule :

HA (III)

A ayant la signification donnée ci-dessus :

2. Procédé selon la revendication 1, caractérisé en ce que l'on prépare un composé de formule I dans laquelle A est un groupe pyrrolidino.

3. Procédé selon la revendication 1, caractérisé en ce que l'on prépare un composé de formule I dans laquelle A est choisi parmi un groupe pipéridino, 3-méthylpipéridino, 3-hydroxyméthylpipéridino.

4. Procédé selon la revendication 1, caractérisé en ce que l'on prépare la 4-pyrrolidino-1-(2-thiényl) butanone ou ses sels d'addition avec des acides pharmaceutiquement acceptables.

5. Procédé de préparation d'une composition thérapeutique, caractérisé en ce que l'on met un composé tel que défini dans l'une quelconque des revendications 1 à 4, sous une forme pharmaceutiquement acceptable.

6. Utilisation d'un composé tel que défini dans l'une quelconque des revendications 1 à 4 pour la fabrication d'un médicament à activité vasodilatatrice.

7. Variante d'un procédé selon la revendication 1, comprenant la réaction d'un nitrile de formule

$NC-(CH_2)_3 - A$ (IV)

avec un dérivé lithié du bromo-2 ou 3-thiophène, puis l'hydrolyse du produit formé.

**Claims**
**Claims for the following Contracting States : AT, BE, CH, DE, DK, FR, GB, GR, IT, LI, LU, NL, SE**

1. Compounds of formula:

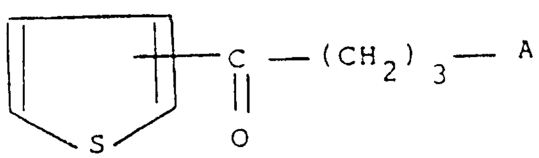

(I)

wherein A is selected from the pyrrolidino, piperidino, morpholino, 1-imidazolyl, hexamethyleneimino and 1-piperazinyl groups, these groups either being unsubstituted or having 1 or 2 substituents selected from the groups $C_{1-3}$ alkyl or $C_{1-3}$ hydroxyalkyl, and the addition salts of these compounds with pharmaceutically acceptable acids.

2. Compounds according to claim 1, wherein A is a pyrrolidino group.

3. Compounds according to claim 1, wherein A is selected from a piperidino, 3-methylpiperidino or 3-hydroxymethylpiperidino group.

4. Compounds according to claim 1 selected from 4-pyrrolidino-1-(2-thienyl)butanone and the addition salts thereof with pharmaceutically acceptable acids.

5. Therapeutic composition containing as active principle a compound according to any one of claims 1 to 4.

6. Use of a compound according to any one of claims 1 to 4 for producing a drug having a vasodilatory activity.

**Claims for the following Contracting State : ES**

1. Process for preparing compounds of formula:

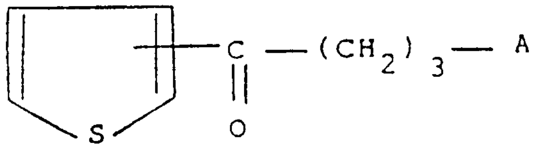

(I)

wherein A is selected from the pyrrolidino, piperidino, morpholino, 1-imidazolyl, hexamethyleneimino and 1-piperazinyl groups, these groups either being unsubstituted or having 1 or 2 substituents selected from the groups $C_{1-3}$ alkyl or $C_{1-3}$ hydroxyalkyl groups, and the addition salts of these compounds with pharmaceutically acceptable acids,
comprising condensing a chloro derivative of formula:

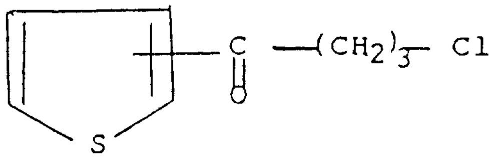

(II)

with a cyclic amine of formula:

HA (III)

wherein A is as hereinbefore defined.

2. Process according to claim 1, characterised in that a compound of formula I is prepared wherein A is a pyrrolidino group.

3. Process according to claim 1, characterised in that a compound of formula I is prepared wherein A is selected from a piperidino, 3-methylpiperidino or 3-hydroxymethylpiperidino group.

4. Process according to claim 1, characterised in that 4-pyrrolidino-1-(2-thienyl)butanone or the addition salts thereof with pharmaceutically acceptable acids is or are prepared.

5. Process for preparing a therapeutic composition, characterised in that a compound as defined in any one of claims 1 to 4 is put into a pharmaceutically acceptable form.

6. Use of a compound as defined in any one of claims 1 to 4 for the preparation of a drug having a vasodilatory activity.

7. Variant of a process according to claim 1, comprising reacting a nitrile of formula

$NC-(CH_2)_3 - A$ (IV)

with a lithium derivative of bromo-2- or -3-thiophene, then hydrolysing the resulting product.

**Patentansprüche**

**Patentansprüche für folgende Vertragsstaaten : AT, BE, CH, DE, DK, FR, GB, GR, IT, LI, LU, NL, SE**

**1.** Verbindungen der Formel

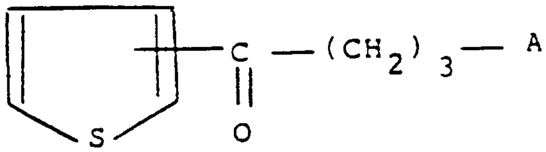

(I)

in der A ausgewählt ist aus den Gruppen Pyrrolidino, Piperidino, Morpholino, 1-Imidazolyl, Hexamethylen-imino, 1-Piperazinyl, wobei diese Gruppen nicht substituiert sind oder einen oder zwei Substituenten besitzen können, die aus den $C_1$-$C_3$-Alkyl- und $C_1$-$C_3$-Hydroxyalkyl-Gruppen ausgewählt sind, und die Additionssalze dieser Verbindungen mit pharmazeutisch akzeptablen Säuren.

**2.** Verbindungen nach Anspruch 1, in denen A eine Pyrrolidino-Gruppe ist.

**3.** Verbindungen nach Anspruch 1, in denen A aus einer Gruppe Piperidino, 3-Methylpiperidino, 3-Hydroxymethylpiperidino ausgewählt ist.

**4.** Verbindungen nach Anspruch 1, die ausgewählt sind aus 4-Pyrrolidino-1-(2-thienyl)-butanon, und ihre Additionssalze mit pharmazeutisch akzeptablen Säuren.

**5.** Therapeutische Zusammensetzung, die als Wirkstoff eine Verbindung nach einem der Ansprüche 1 bis 4 enthält.

**6.** Verwendung einer Verbindung nach einem der Ansprüche 1 bis 4 zur Herstellung eines Medikaments mit gefäßerweiternder Wirkung.

**Patentansprüche für folgenden Vertragsstaat : ES**

**1.** Verfahren zur Herstellung von Verbindungen der Formel

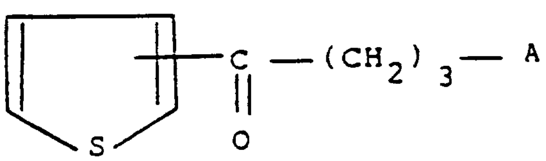

(I)

in der A ausgewählt ist aus den Gruppen Pyrrolidino, Piperidino, Morpholino, 1-Imidazolyl, Hexamethylen-imino, 1-Piperazinyl, wobei diese Gruppen nicht substituiert sind oder einen oder zwei Substituenten aufweisen können, die aus den Gruppen $C_1$-$C_3$-Alkyl- und $C_1$-$C_3$-Hydroxyalkyl ausgewählt sind, und die Additionssalze dieser Verbindungen mit pharmazeutisch akzeptablen Säuren, umfassend die Kondensierung eines Chlorderivats der Formel

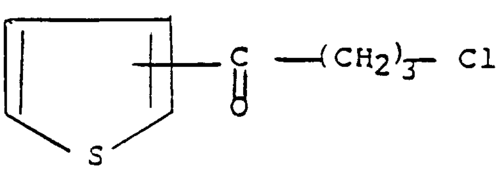

(II)

mit einem zyklischen Amin der Formel:

HA (III)

worin A die oben angegebene Bedeutung hat.

**2.** Verbindungen nach Anspruch 1, dadurch gekennzeichnet, daß man eine Verbindung der Formel I herstellt, in der A eine Pyrrolidino-Gruppe ist.

**3.** Verfahren nach Anspruch 1, dadurch gekennzeichnet, daß man eine Verbindung der Formel I herstellt, in der A aus einer Gruppe Piperidino, 3-Methylpiperidino, 3-Hydroxymethylpiperidino ausgewählt ist.

**4.** Verfahren nach Anspruch 1, dadurch gekennzeichnet, daß man 4-Pyrrolidino-1-(2-thienyl)-butanon oder seine Additionssalze mit pharmazeutisch akzeptablen Säuren herstellt.

**5.** Verfahren zur Herstellung einer therapeutischen Zusammensetzung, dadurch gekennzeichnet, daß man eine Verbindung gemäß einem der Ansprüche 1 bis 4 in eine pharmazeutisch akzeptable Form bringt.

**6.** Verwendung einer Verbindung nach einem der Ansprüche 1 bis 4 zur Herstellung eines Medikaments mit gefäßerweiternder Wirkung.

**7.** Abwandlung eines Verfahrens nach Anspruch 1, umfassend die Reaktion eines Nitrils der Formel

$NC\text{-}(CH_2)_3 - A$ (IV)

mit einem Lithiumderivat von Brom-2 oder 3-Thiophen und dann die Hydrolyse des gebildeten Produkts.